# EUROPEAN PATENT SPECIFICATION

(11) **EP 4 314 823 B1**
(45) Date of publication and mention of the grant of the patent: **09.12.2026**
(21) Application number: 22717004.0
(22) Date of filing: 21.03.2022
(51) Int. Cl.: G01N 33/543, G01N 33/68, G01N 33/94, C12Q 1/34

(54) **BIOLOGICAL SAMPLE PREPARATION AND ANALYSIS**
VORBEREITUNG UND ANALYSE BIOLOGISCHER PROBEN
PRÉPARATION ET ANALYSE D'ECHANTILLONS BIOLOGIQUES

(30) Priority: 23.03.2021 US 202163164870 P
(43) Date of publication of application: 07.02.2024
(73) Proprietor: DH Technologies Development Pte. Ltd., Singapore 739256 (SG)
(72) Inventor: STELLA, Aaron, Concord, Ontario L4K4V8 (CA); LIU, Chang, Concord, Ontario L4K4V8 (CA); LIU, Yuguan, Concord, Ontario L4K4V8 (CA); PURKAYASTHA, Subhasish, Concord, Ontario L4K4V8 (CA)
(74) Representative: Maiwald GmbH
(86) International application number: PCT/IB2022/052566
(87) International publication number: WO 2022/201000

(56) References cited:
- WO-A1-2015/077660
- WO-A1-2019/168843
- US-A1- 2016 231 341
- US-A1- 2017 138 940
- US-A9- 2019 137 484
- OLSEN J. V. ET AL: "Trypsin cleaves...", 1 June 2004 (2004-06-01), XP093330122, Retrieved from the Internet <URL:https://www.sciencedirect.com/science/article/pii/S1535947620338779>
- RADISKY E. S. ET AL: "Insights into the serine ....", 2 May 2006 (2006-05-02), XP093330123, Retrieved from the Internet <URL:https://www.pnas.org/doi/epdf/10.1073/pnas.0601910103>
- CORREA SONALI ET AL: "Stabilization of b-Glucuronidase by Immobilization in Magnetic-Silica Hybrid Supports", CATALYSTS, vol. 10, no. 6, 13 June 2020 (2020-06-13), CH, pages 669, XP093330375, ISSN: 2073-4344, DOI: 10.3390/catal10060669
- MIAO A ET AL: "Liquid-chromatographic and mass-spectrometric identification of lens proteins using microwave-assisted digestion with trypsin-immobilized magnetic nanoparticles", BIOCHEMICAL AND BIOPHYSICAL RESEARCH COMMUNICATIONS, ELSEVIER, AMSTERDAM NL, vol. 380, no. 3, 13 March 2009 (2009-03-13), pages 603 - 608, XP026422262, ISSN: 0006-291X, [retrieved on 20090127], DOI: 10.1016/J.BBRC.2009.01.132
- HU ZHENGYAN ET AL: "The on-bead digestion of protein corona on nanoparticles by trypsin immobilized on the magnetic nanoparticle", JOURNAL OF CHROMATOGRAPHY A, ELSEVIER, AMSTERDAM, NL, vol. 1334, 5 February 2014 (2014-02-05), pages 55 - 63, XP028624797, ISSN: 0021-9673, DOI: 10.1016/J.CHROMA.2014.01.077
- JAGERDEO ESHWAR ET AL: "Rapid screening for drugs of abuse in biological fluids by ultra high performance liquid chromatography/Orbitrap mass spectrometry", JOURNAL OF CHROMATOGRAPHY B, ELSEVIER, AMSTERDAM, NL, vol. 1027, 10 May 2016 (2016-05-10), pages 11 - 18, XP029624424, ISSN: 1570-0232, DOI: 10.1016/J.JCHROMB.2016.05.010
- SUKHANOVA I I ET AL: "Magnetic separation as a new method for the extraction of small molecules from biological fluids of humans", JOURNAL OF ANALYTICAL CHEMISTRY, KLUWER ACADEMIC PUBLISHERS-PLENUM PUBLISHERS, NE, vol. 66, no. 9, 25 August 2011 (2011-08-25), pages 807 - 814, XP019944107, ISSN: 1608-3199, DOI: 10.1134/S1061934811090164
- PONT LAURA ET AL: "Comparison of magnetic bead surface functionalities for the immunopurification of growth hormone-releasing hormones prior to liquid chromatography-high resolution mass spectrometry", JOURNAL OF CHROMATOGRAPHY A, ELSEVIER, AMSTERDAM, NL, vol. 1631, 17 September 2020 (2020-09-17), XP086297125, ISSN: 0021-9673, [retrieved on 20200917], DOI: 10.1016/J.CHROMA.2020.461548
- CHALET CLÉMENT ET AL: "Identification of phase-II metabolites of flavonoids by liquid chromatography-ion-mobility spectrometry-mass spectrometry", ANALYTICAL AND BIOANALYTICAL CHEMISTRY, SPRINGER BERLIN HEIDELBERG, BERLIN/HEIDELBERG, vol. 410, no. 2, 22 November 2017 (2017-11-22), pages 471 - 482, XP036395855, ISSN: 1618-2642, [retrieved on 20171122], DOI: 10.1007/S00216-017-0737-4

## Description

### BACKGROUND

Liquid samples including biological materials and bodily fluids, can often provide critical analytical information. However, many of these liquid samples include a variety of proteins that may hinder downstream analysis of small molecules. Additionaly target analytes may undergo metabolism including glucuronidation, resulting in complexes that may prove challeding the analyze directly. To achieve a more accurate analysis of liquid samples, proper sample preparation is critical. Typical sample preparation methods may require significant time and specific conditions and are not easily automated.
"Liquid-chromatographic and mass-spectrometric identification of lens proteins using microwave-assisted digestion with trypsin-immobilized magnetic nanoparticles" by Miao et al. relates to a method combining trypsin-immobilized magnetic nanoparticles (TIMNs) and microwave-assisted protein digestion to study the proteins of human lens tissue.
"The on-bead digestion of protein corona on nanoparticles by trypsin immobilized on the magnetic nanoparticle" by Hu et al. relates to proteins interacting with nanoparticles.
US 2016/0231341 relates to a method for the determination of multiple drugs and their metabolites.
"Rapid screening for drugs of abuse in biological fluids by ultra high performance liquid chromatography/Orbitrap mass spectrometry" by E. Jagerdeo & J.E. Schaff relates to a High Resolution Mass Spectrometric method.
"Magnetic Separation as a New Method for the Extraction of Small Molecules from Biological Fluids of Humans" by Sukhanova et al. relates to the extraction of doping substances from urine.
"Comparison of magnetic bead surface functionalities for the immunopurification of growth hormone-releasing hormones prior to liquid chromatography-high resolution mass spectrometry" by Pont et al. relates to the suitability of magnetic beads with different supports, binding capacities and affinity chemistries.
US 2017/0138940 A1 relates to a method for isolating trace components selected from various groups and substances.
US 2019/0137484 A9 relates to magnetic and non-magnetic microparticle binding surfaces.
WO 2019/168843 A1 relates to the field of mass spectrometry analysis.
"Stabilization of b-Glucuronidase by Immobilizationin Magnetic-Silica Hybrid Supports" by Correa et al. relates to mmobilization strategies for Patella vulgate β-Glucuronidase.

### SUMMARY

The inventors have recognized the need for a sample preparation method that efficiently extracts protein from liquid samples before downstream analysis. In particular, a sample preparation method that employs both hydrolysis and magnetic bead purification.

One aspect of the disclosure relates to a method of preparing a liquid sample, comprising the steps of treating the liquid sample with a hydrolysis enzyme, hydrolyzing the liquid sample to prepare a hydrolysate, and purifying the hydrolysate with magnetic based purification. In one aspect, the liquid sample is a biological sample. In another aspect, the biological sample is selected from the group consisting of urine, blood, oral fluid, and plasma. In yet another aspect, the hydrolysis enzyme is bound to a magnetic bead or a magnetic particle.

In one aspect, the magnetic based purification utilizes magnetic beads or magnetic particles. In a further aspect, the magnetic based purification further comprises the steps of precipitating proteins and any excess hydrolysis enzyme in the hydrolysate with a precipitating reagent, incubating the magnetic beads or magnetic particles with the precipitated proteins for a time sufficient to produce a suspension, and magnetically separating the magnetic beads or magnetic particles from the suspension to produce a supernatant. In one aspect, the precipitating reagent is selected from the group consisting of zinc salts, zinc sulfate, glycols, alcohols, acids, sulfates, acids, acetonitrile, and combinations thereof. In a further aspect, the precipitating reagent is 0.4M zinc sulfate.

In some aspects, the magnetic based purification is based on immunopurification or affinity purification. In another aspect, the magnetic bead or magnetic particle comprises a monoclonal antibody, a polyclonal antibody, a synthetic antibody mimic, an aptamer, an affimer, DARPins, or oligonucleotides or peptides that bind to specific targets with high affinity. In some aspects, the magnetic bead or magnetic particle comprises streptavidin, and the hydrolysis enzyme comprises biotin. In some aspects, the magnetic based purification is based on ion-exchange. In yet another aspect, the magnetic beads or magnetic particles have a surface coating selected from the group consisting of dextran, sodium carboxylate, phosphate, diphosphate, polyacrylic acid, oleic acid, silica, 2-hydroxypropyl trimethylammonium chloride, polyethylenimine, diethylaminoethyl cellulose, poly(4-vinylpyridine), and sodium sulfonate.

In some aspects, the liquid sample further comprises an internal standard. In another aspect, the hydrolysis enzyme is capable of hydrolyzing glycosidic linkages. In yet another aspect, the hydrolysis enzyme is β-glucuronidase, trypsin, chymotrypsin, a protease, LysC, LysN, AspN, GluC, ArgC, pronase, pepsin, prolidase. In a further aspect, the hydrolysis enzyme is capable of hydrolyzing one or more linkages in a metabolite such as, for example, an opioid metabolite (e.g., codeine-6-glucuronide and morphine-6-glucuronide linkages).

In some aspects, the hydrolyzing step is performed for up to about 20 minutes. In some aspects, this step is performed for about 10 minutes to about 20 minutes. In a further aspect, the hydrolyzing step is performed for about 15 minutes. In another aspect, the hydrolyzing step is performed at a temperature of up to 60 °C. In some aspects, this step is performed at about 50 °C to about 60 °C. In yet another aspect, the aqueous biological sample is hydrolyzed at a temperature of about 55 °C.

In some aspects, the incubating further comprises mixing, shaking, or vortexing the magnetic beads or magnetic particles with the hydrolysate.

In some aspects, the method further comprises aliquoting the supernatant. In yet another aspect, the aliquoted supernatant is separated and/or enriched using a chromatography instrument, microflow, or solid phase extraction.. In some aspects, the chromatography instrument is a high performance liquid chromatography (HPLC) instrument or an ultra high performance liquid chromatography instrument (UPLC). In a further aspect, the aliquoted supernatant is separated and/or enriched using a trap-and-elute workflow.

In some aspects, the aliquoted supernatant is acoustically injected into an open port interface and transferred to an ionization source or directly injected into an ionization source. In another aspect, the ionized supernatant is analyzed with a mass spectrometer. In some aspects, ions of interest are selected from the ionized supernatant using differential mobility spectrometry prior to analyzing the ionized supernatant with a mass spectrometer.

In some aspects, the method improves the efficiency of the liquid sample hydrolysis or improves the liquid sample hydrolysate quality. In another aspect, the improved quality of the hydrolysate includes a reduction in carryover. In yet another aspect, the method is used in an automated and/or continuous process of preparing a liquid sample.

In some aspects, the method is used to prepare a liquid sample for clinical analysis.. In some aspects, the clinical analysis is used to screen for drugs of abuse. In a further aspect, the screened drugs of abuse are selected from the group consisting of amphetamines, methamphetamines, benzodiazepines, barbiturates, marijuana, cocaine, PCP, methadone, and opioids (narcotics).

One aspect of the disclosure relates to a kit for preparing a liquid sample, wherein the kit comprises:
a hydrolysis enzyme selected from the group consisting of β-glucuronidase, trypsin, chymotrypsin, proteases, LysC, LysN, AspN, GluC, ArgC, pronase, pepsin, prolidase, and a biotinylated enzyme, and
magnetic beads or magnetic particles, wherein the magnetic beads or magnetic particles comprise a monoclonal antibody, a polyclonal antibody, streptavidin, or have a surface coating selected from the group consisting of dextran, sodium carboxylate, phosphate, diphosphate, polyacrylic acid, oleic acid, silica, 2-hydroxypropyl trimethylammonium chloride, polyethylenimine, diethylaminoethyl cellulose, poly(4-vinylpyridine), and sodium sulfonate. In some aspects, the kit may include one or more internal standards, and/or a liquid chromatography column and/or one or more solvents to be used as mobile phases, one or more calibrant solutions, and instructions for use.

Other aspects and features of the present disclosure will become apparent to those ordinarily skilled in the art upon review of the following description of specific embodiments of the disclosure in conjunction with the accompanying figures.

### BRIEF DESCRIPTION OF THE FIGURES

Embodiments of the present disclosure will now be described, by way of example only, with reference to the attached Figures, wherein:
FIGs. 1A-1E illustrate isobaric chromatograms for drugs of abuse according to one embodiment of the disclosure.
FIG. 1A illustrates an embodiment of the disclosure where peak identities are morphine, hydromorphone, norcodeine, and norhydrocodone.
FIG. 1B illustrates an embodiment of the disclosure where peak identities are codeine and hydrocodone.
FIG. 1C illustrates an embodiment of the disclosure where peak identities are oxymorphone, dihydrocodeine, and noroxycodone.
FIG. 1D illustrates an embodiment of the disclosure where peak identities are 6-Monoacetylmorphine (6-MAM), a unique metabolite of heroin, and naloxone.
FIG. 1E illustrates an embodiment of the disclosure where peak identities are norclozapine and flunitrazepam.

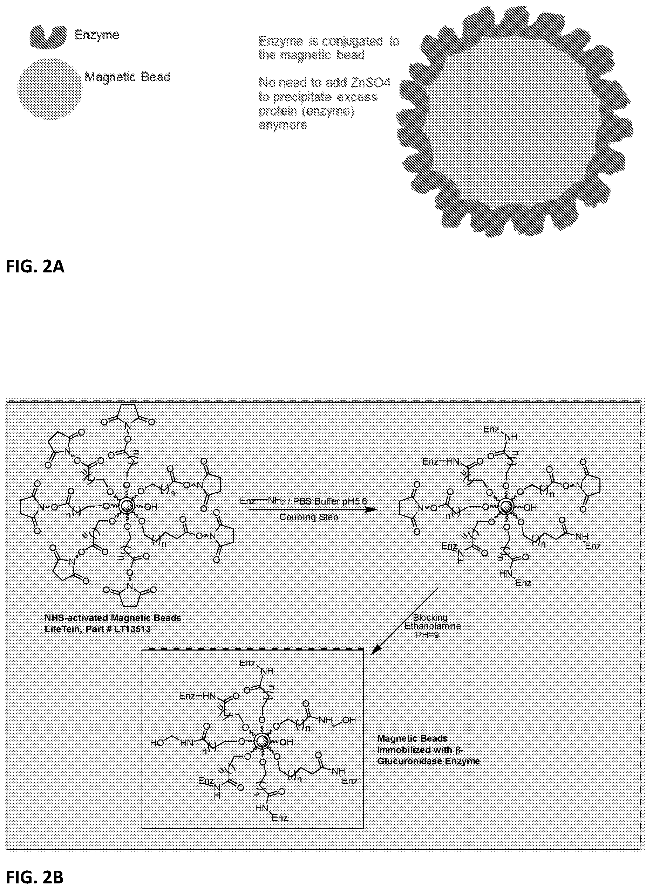

FIG. 2A illustrates an enzyme conjugated to a magnetic bead.
FIG. 2B illustrates the chemical immobilization of β-Glucuronidase to a magnetic bead.
FIG. 3 is a graphical representation of the pressure tracings from liquid samples hydrolyzed according to an embodiment.

### DETAILED DESCRIPTION

It is to be understood that this disclosure is not limited to the particular methodology, protocols, and reagents described herein and as such may vary. It is also to be understood that the terminology used herein is for the purpose of describing particular embodiments only and is not intended to limit the scope of the present disclosure or the appended claims.

As used herein and in the appended claims, the singular forms "a," "an," and "the" include plural reference unless the context clearly indicates otherwise.

The term "about" is used in connection with a numerical value throughout the specification and the claims denote an interval of accuracy, familiar and acceptable to a person skilled in the art. In general, such an interval of accuracy is +/-10%.

Unless defined otherwise, all technical and scientific terms used herein have the same meaning as commonly understood to one of ordinary skill in the art to which this disclosure belongs.

Embodiments of the disclosure include methods of preparing a liquid sample. The liquid sample may be a biological sample. Biological samples may be biological fluids, which may include, but are not limited to, blood, plasma, serum, oral fluid, or other bodily fluids or excretions, such as but not limited to saliva, urine, cerebrospinal fluid, lacrimal fluid, perspiration, gastrointestinal fluid, amniotic fluid, mucosal fluid, pleural fluid, sebaceous oil, exhaled breath, and the like.

In an embodiment, the method of preparing a liquid sample includes hydrolyzing the liquid samples. In some embodiments, an internal standard is added before hydrolysis, but it can be appreciated by one of ordinary skill that the internal standard may be added after hydrolysis is completed.

Hydrolysis can be chemical or enzymatic. Enzymatic hydrolysis is a process where peptide bonds in proteins are hydrolyzed using enzymes, such as proteases, peptidases, or peptide hydrolases. Proteases can be either exopeptidases, which act near the end of a polypeptide chain and include, for example, aminopeptidases and dipeptidyl peptidases, or endopeptidases, which act on nonterminal peptide bonds and include, for example, serine proteases, cysteine proteases, aspartic acid proteases, and metallo endopeptidases.

In some embodiments, the method includes treating the liquid sample with a hydrolysis enzyme to prepare a hydrolysate. Suitable hydrolysis enzymes include, but are not limited to, β-glucuronidase, trypsin, chymotrypsin, a protease, LysC, LysN, AspN, GluC, ArgC, pronase, pepsin, and prolidase. Suitable hydrolysis enzymes also include those that are capable of hydrolyzing glycosidic linkages, such as those formed during metabolic processes. Non-limiting examples of these glycosidic linkages include codeine-6-glucuronide and morphine-6-glucuronide linkages.

The hydrolysis enzyme may be incubated with the liquid sample for a suitable amount of time. Suitable amounts of time may be dependent on the hydrolysis enzyme used and the liquid sample being hydrolyzed. Non-limiting examples of hydrolysis incubation times include about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, or about 20 minutes. In some methods, hydrolysis incubation times of greater than about 20 minutes may be employed.

The hydrolysis enzyme may be incubated with the liquid sample at a suitable temperature. Suitable temperatures may be dependent on the hydrolysis enzyme used and the liquid sample being hydrolyzed. Non-limiting examples of hydrolysis temperatures include about 45 °C, about 46 °C, about 47 °C, about 48 °C, about 49 °C, about 50 °C, about 51 °C, about 52 °C, about 53 °C, about 54 °C, about 55 °C, about 56 °C, about 57 °C, about 58 °C, about 59 °C, or about 60 °C. In an embodiment, the hydrolysis enzyme is incubated with the biological sample for about 20 minutes at about 55 °C.

In an embodiment, the method of preparing a liquid sample includes purifying the hydrolysate with magnetic based purification. In some embodiments, the magnetic based purification utilizes magnetic bead or magnetic particles. Magnetic beads or magnetic particles are typically nanoparticles or microparticles that have paramagnetic properties. Magnetic beads or magnetic particles are typically hydrophilic and disperse easily in aqueous solutions. The surface coating and/or chemistry of the magnetic beads or magnetic particles allow various biomolecules such as proteins, peptides, and nucleic acids to bind to the magnetic beads or magnetic particles. Once a biomolecule of interest is bound to a magnetic bead or magnetic particle, magnetic separation is employed to the magnetic beads or magnetic particles from a suspension by applying a magnetic force.

In an embodiment, the hydrolysis enzyme is bound to magnetic beads or magnetic particles. In an embodiment, the hydrolysis enzyme is chemically bonded to the magnetic bead or magnetic particle. In an exemplary embodiment, subjecting the liquid sample to hydrolysis in the presence of the immobilized hydrolysis enzyme generates a hydrolyzed liquid sample that can be acoustically ejected in an open port interface and transferred to an ionization source or directly injected into an ionization source.

In an embodiment, the magnetic based purification is based on immunopurification or affinity purification. In these embodiments, the magnetic beads or magnetic particles may comprise a monoclonal antibody, a polyclonal antibody, a synthetic antibody mimic, an aptamer, an affimer, DARPins, or oligonucleotides or peptides that bind to specific targets with high affinity. In an embodiment, the magnetic based purification employs the streptavidin-biotin system, where the magnetic bead or magnetic particle comprises streptavidin, and the hydrolysis enzyme comprises biotin. In an exemplary embodiment, the liquid sample is hydrolyzed using the biotinylated enzyme. After hydrolysis is complete, the biotinylated enzyme is affinity captured using a streptavidin-coated magnetic bead or magnetic particle. The resulting hydrolyzed liquid sample can be acoustically ejected in an open port interface and transferred to an ionization source or directly injected into an ionization source.

In an embodiment, the magnetic bead purification is based on ion-exchange. In these embodiments, the magnetic beads or magnetic particles have a surface coating selected from the group consisting of dextran, sodium carboxylate, phosphate, diphosphate, polyacrylic acid, oleic acid, silica, 2-hydroxypropyl trimethylammonium chloride, polyethylenimine, diethylaminoethyl cellulose, poly(4-vinylpyridine), and sodium sulfonate.

In an embodiment, the magnetic bead purification step comprises precipitating the proteins in the hydrolysate and/or and any excess hydrolysis enzyme. Non-limiting examples of precipitation methods include changes in temperature, pH, and/or salt concentrations or the addition of a precipitating reagent. *See, e.g.,* Metz, Clyde, Chemistry: Inorganic Qualitative Analysis in the Laboratory, 1980. In an embodiment, the proteins in the hydrolysate are precipitated using a precipitating reagent. Non-limiting examples of precipitating reagents include zinc salts, zinc sulfate, glycols, alcohols, acids, sulfates, acids, acetonitrile. Precipitating reagents may be used alone or in combination with other precipitating reagents or other organic solvents. In one embodiment, the precipitating reagent is 0.4M zinc sulfate.

In an embodiment, the magnetic bead purification step also comprises incubating the magnetic beads or magnetic particles with the precipitated proteins for a time sufficient to produce a suspension. Suitable amounts of time may be dependent on the type of magnetic bead purification being employed. In some embodiments, the time sufficient is less than 10 minutes, alternatively less than 9 minutes, alternatively less than 8 minutes, alternatively less than 7 minutes, alternatively less than 6 minutes, alternatively less than 5 minutes, alternatively less than 4 minutes, alternatively less than 3 minutes, alternatively less than 2 minutes, alternatively less than 1 minute.

In an embodiment, the magnetic bead purification step also comprises magnetically separating the magnetic beads or magnetic particles from the suspension to produce a supernatant. Magnetic separation may include applying a magnetic field to the magnetic beads or magnetic particles, which will draw the magnetic beads or magnetic particles toward the side wall of a sample container. The magnetic field may be applied using a magnetic rack. This allows for the removal of a biomolecule of interest bound to the magnetic beads or magnetic particles from the suspension and results in a supernatant that can be aliquoted and analyzed.

In an embodiment of the disclosure, an analyzer may be used to analyze aliquoted supernatant. The term "analyzer" may include any suitable instrument capable of analyzing a sample such as a biological sample. Examples of analyzers include chromatography instruments, mass spectrometers, immunoanalyzers, hematology analyzers, microbiology analyzers, and/or molecular biology analyzers.

In an embodiment, the aliquoted supernatant is separated and/or enriched using a chromatography instrument, microflow, or solid phase extraction. Examples of chromatography instruments, include, but is not limited to, a liquid chromatography instrument such as a highperformance liquid chromatography (HPLC) instrument or an ultra-high performance chromatography (UHPLC) instrument.

In an embodiment, the aliquoted supernatant is analyzed using a mass spectrometer. The aliquoted supernatant may be introduced into the mass spectrometer with additional separation or without additional separation. If separation is not required, the aliquoted supernatant may be acoustically injected into an open port interface and transferred to an ionization source or directly injected into an ionization source. Separation and/or enrichment done prior to mass spectrometry analysis may include, but is not limited to, liquid chromatography, microflow, and solid phase extraction. In some embodiment, a trap-and-elute workflow may be used.

In an embodiment, ions of interest are selected from the ionized supernatant using differential mobility spectrometry prior to analyzing the ionized supernatant with a mass spectrometer. Differential mobility spectrometry enables the separation of coeluting compounds, isobaric compounds, isomeric compounds, constitutional isomers, or diastereomers.

In an embodiment, the method is used to prepare a liquid sample for clinical analysis. The clinical analysis can be used to screen for drugs of abuse. Non-limiting examples of drugs of abuse include amphetamines, methamphetamines, benzodiazepines, barbiturates, marijuana, cocaine, PCP, methadone, and opioids (narcotics). In an embodiment, the clinical analysis is a clinical urine test or a urinalysis, and is the analysis is used to screen for drugs of abuse. Urine is a common biological sample used in testing for drugs of abuse. A urinalysis or clinical urine test can detect the presence of a drug of abuse after the drug effects have worn off.

The disclosure includes a kit for preparing a liquid sample for analysis. The kit comprises a hydrolysis enzyme. Non-limiting examples of the hydrolysis enzyme include β-glucuronidase, trypsin, chymotrypsin, a protease, LysC, LysN, AspN, GluC, ArgC, pronase, pepsin, prolidase, or a biotinylated enzyme. The kit also includes magnetic beads or magnetic particles. In some embodiments, the magnetic bead or magnetic particles may comprise a monoclonal antibody, a polyclonal antibody, streptavidin, or have a surface coating selected from the group consisting of dextran, sodium carboxylate, phosphate, diphosphate, polyacrylic acid, oleic acid, silica, 2-hydroxypropyl trimethylammonium chloride, polyethylenimine, diethylaminoethyl cellulose, poly(4-vinylpyridine), and sodium sulfonate. The kit may further include one or more internal standards. In some embodiments, the internal standard may be added in a constant amount to samples, the blank, and calibration standards. The internal standard may be a compound that is highly similar to an analyte of interest. The kit may also include a liquid chromatography column. The liquid chromatography column may be a reversed-phase chromatography column. Non-limiting examples of reversed-phase chromatography columns include C18-bonded silica, C8-bonded silica, pure silica, cyano-bonded silica, and phenyl-bonded silica. The kit may also include one or more solvents. Non-limiting examples of suitable chromatography solvents include organic solvents, such as acetonitrile, methanol, and propanol. To improve the chromatographic peak shape, acids, such as formic acid, triflouroacetic acid, or acetic acid, may be included. These acids also provide a source of protons in reverse phase LC/MS applications. The kit may also include one or more calibrant or calibration solution. The calibrant or calibration solution may be used to calibrate an analyzer. The kit may also include instructions for use.

### EXAMPLES

### Example 1: Exemplary sample preparation and analysis method

Urine samples can be collected according to the SAMHSA guidelines (Substance Abuse and Mental Health Services Administration Center for Substance Abuse Prevention), available at https://www.samhsa.gov/sites/default/files/specimen-collection-handbook-2014.pdf. Urine specimens are typically submitted to certified laboratories within 24 hours after collection.

A 30 µL aliquot of an internal standard, 80 µL of water, 40 µL of a hydrolysis enzyme, and 20 µL of urine sample are added to a plastic reaction vessel. The urine sample is mixed via vortexing and is subsequently hydrolyzed by a fast-acting enzyme that is proven capable of hydrolyzing glycosidic linkages, including codeine-6-glucuronide and morphine-6-glucuronide, in 15 minutes at 55 °C. Magnetic beads (1 mg/mL) in the amount of 25 µL, and 105 µL of water, and 30 µL of 0.4M zinc sulfate are then added to the reaction milieu. After vortexing briefly, a magnetic rack is then used to pull the magnetic beads with the precipitated proteins to one side of the reaction vessel, leaving behind a very clear supernatant that contains the analyte(s) of interest. This supernatant can then be easily aliquoted into an HPLC vial or directly injected into a mass spectrometer.

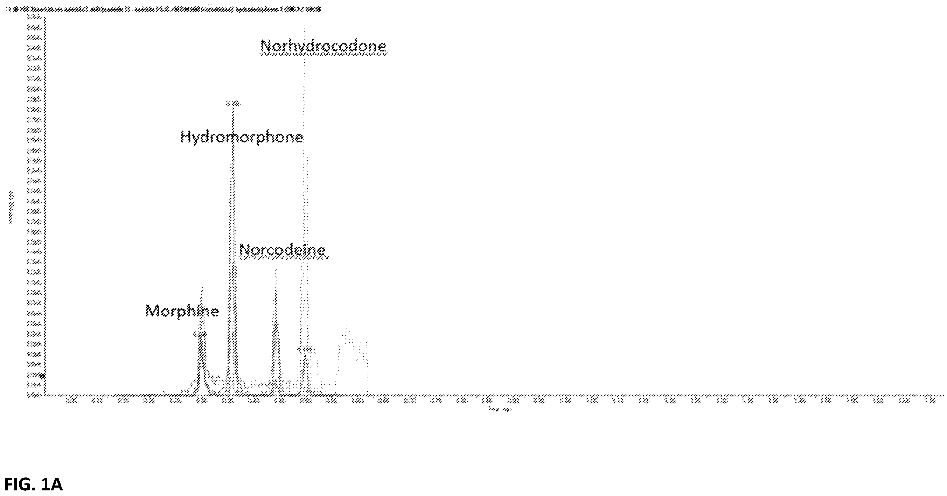

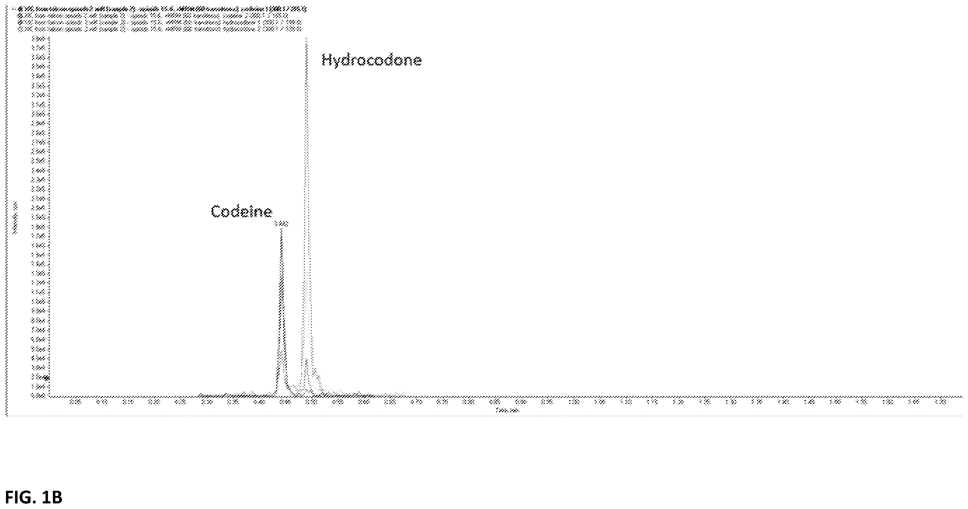

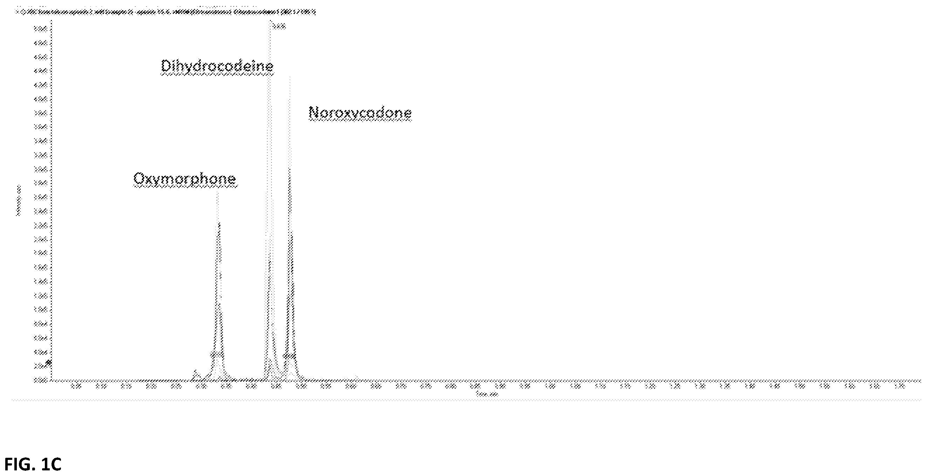

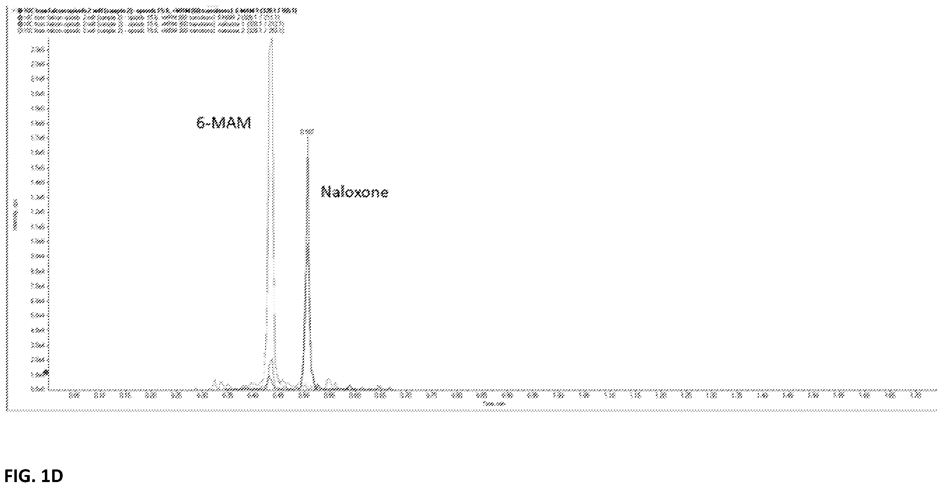

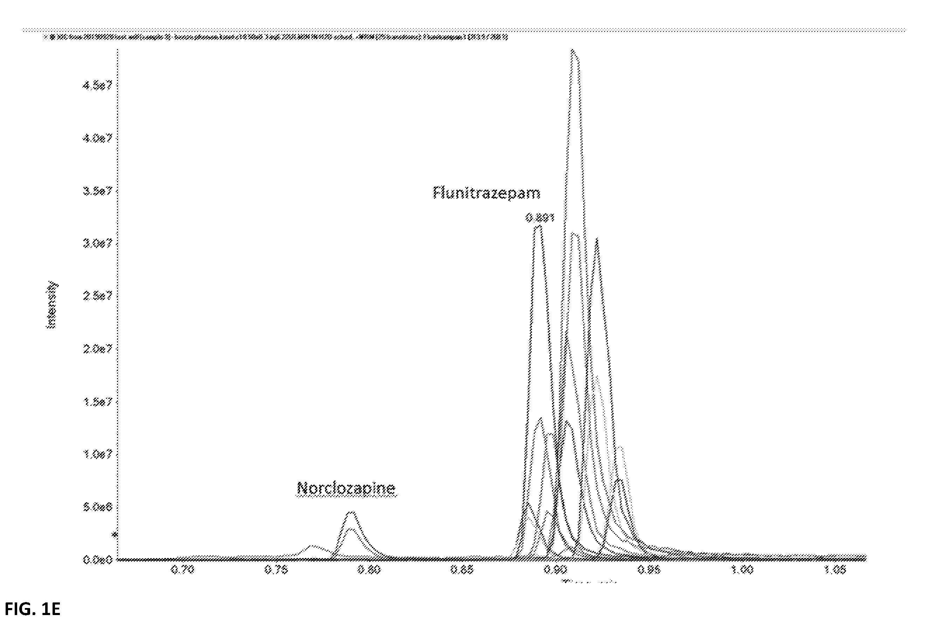

Chromatograms of selected samples are illustrated in FIGs. 1A-1E and identify the presence of morphine, hydromorphone, norcodeine, norhydrocodone (FIG. 1A), codeine and hydrocodone (FIG. 1B), oxymorphone, dihydrocodeine, and noroxycodone (FIG. 1C), 6-Monoacetylmorphine (6-MAM) and naloxone (FIG. 1D) and norclozapine and flunitrazepam (FIG. 1E).

### Example 2: On-bead hydrolysis of liquid samples

Calibration curves and quality control samples for morphine and codeine were run using both a traditional hydrolysis method and an on-bead hydrolysis method. The study samples were urine samples were spiked with morphine-6-glucuronide and codeine-6-glucuronide. Table 1 illustrates the target concentrations.

**Table 1.**

| **Hyd Control** | Gluc, ng/mL | Free, ng/mL |
|---|---|---|
| Codeine-6-Gluc | 15000 | 9444 |
| Morphine-3-Gluc | 15000 | 9275 |

The test samples were run using the traditional method, with and without adding a hydrolysis enzyme. The test samples were also run using the hydrolysis enzyme-on-bead method. The results from incubating these samples at 55°C for 15 and 60 minutes are shown in Table 2.

**Table 2.**

| **55 °C, 15 and 60 min., 900 rpm agitation** | | | | |
|---|---|---|---|---|
| **On Bead Method** | **Morphine** | **Accuracy (%)** | **Codeine** | **Accuracy (%)** |
| On Bead Hydrolysis 1-15 min | 13155.63 | 141.84 | 3789.09 | 40.12 |
| On Bead Hydrolysis 2-15 min | 11784.82 | 127.06 | 3502.00 | 37.08 |
| On Bead Hydrolysis 1-60 min | 9966.9 | 107.46 | 6101.15 | 64.6 |
| On Bead Hydrolysis 2-60 min | 10412.75 | 112.27 | 6153.89 | 65.16 |

The glucuronide was cleaved from the morphine-6-glucuronide molecule efficiently enough to produce concentrations within the acceptable range. The glucuronide was also cleaved from the codeine-6-glucuronide molecule, but with less efficiency in a time-dependent manner. A clean sample, suitable for reliable acoustic or direct injections was also produced with the magnetic beads. There is no need to precipitate excess enzyme as the enzyme is conjugated to the beads and would not be injected into a liquid chromatography instrument or ionization source. As a result, a precipitation step during sample preparation was eliminated (saving time and reagent), and a highly-stable and reproducible column pressure profile was achieved throughout the study.

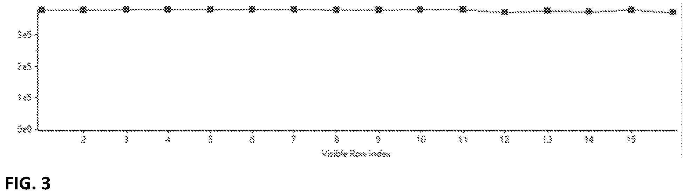

FIG. 3 is a graphical representation of the pressure tracings of each individual sample that was analyzed during the study. Table 3 summarizes the average pressure tracing's area value, its standard deviation, and its very low coefficient of variation value over the 16-sample study.

**Table 3**

| | |
|---|---|
| Mean | 3.78 E+05 |
| SD | 3251.03 |
| % CV | 0.86 |

## Claims

1. A method of preparing a liquid sample, comprising:
treating the liquid sample with a hydrolysis enzyme, which is capable of hydrolyzing glycosidic linkages and is bound to a magnetic bead or a magnetic particle,
hydrolyzing the liquid sample to prepare a hydrolysate, and
purifying the hydrolysate with magnetic based purification.
wherein the magnetic based purification further comprises the steps of:
precipitating proteins with a precipitating reagent,
incubating the magnetic beads or magnetic particles with the precipitated proteins for a time sufficient to produce a suspension, and
magnetically separating the magnetic beads or magnetic particles from the suspension to produce a supernatant.

2. The method of claim 1 wherein the precipitating reagent is selected from the group consisting of zinc salts, zinc sulfate, glycols, alcohols, acids, sulfates, acids, acetonitrile, and combinations thereof.

3. The method of any one of the preceding claims, wherein the magnetic based purification is based on immunopurification, affinity purification or ion exchange.

4. The method of claim 3, wherein the magnetic based purification is based on immunopurification or affinity purification and the magnetic bead or magnetic particle comprises a monoclonal antibody, a polyclonal antibody, a synthetic antibody mimic, an aptamer, an affimer, DARPins, or oligonucleotides or peptides that bind to specific targets with high affinity; or wherein the magnetic based purification is based on ion exchange and the magnetic beads or magnetic particles have a surface coating selected from the group consisting of dextran, sodium carboxylate, phosphate, diphosphate, polyacrylic acid, oleic acid, silica, 2-hydroxypropyl trimethylammonium chloride, polyethylenimine, diethylaminoethyl cellulose, poly(4-vinylpyridine), and sodium sulfonate.

5. The method of any one of the preceding claims, wherein the hydrolysis enzyme is β-glucuronidase.

6. The method of any of the preceding claims, wherein the hydrolysis enzyme is capable of hydrolyzing codeine-6-glucuronide and morphine-6-glucuronide linkages.

7. The method of any one of claims 2-6, further comprising aliquoting the supernatant, wherein (a) the aliquoted supernatant is separated and/or enriched using a chromatography instrument, microflow, solid phase extraction, or a trap-and-elute workflow; and/or (b) the aliquoted supernatant is acoustically injected into an open port interface and transferred to an ionization source or directly injected into an ionization source, and the ionized supernatant is optionally analyzed with a mass spectrometer.

8. The method of claim 7, wherein the aliquoted supernatant is acoustically injected into an open port interface and transferred to an ionization source or directly injected into an ionization source, and ions of interest are selected from the ionized supernatant using differential mobility spectrometry prior to analyzing the ionized supernatant with the mass spectrometer.

9. The method of any of the preceding claims, wherein the method is used to prepare a liquid sample for clinical analysis, and optionally wherein the clinical analysis is used to screen for drugs of abuse.

10. The method of any of the preceding claims, wherein the magnetic beads or magnetic particles comprise a monoclonal antibody, a polyclonal antibody, a synthetic antibody mimic, an aptamer, an affimer, DARPins, or oligonucleotides or peptides that bind to specific targets with high affinity or the magnetic bead or magnetic particle comprises streptavidin and the hydrolysis enzyme comprises biotin.

## Patentansprüche

1. Verfahren zum Herstellen einer flüssigen Probe, umfassend:
Behandeln der flüssigen Probe mit einem Hydrolyseenzym, das glykosidische Bindungen hydrolysieren kann und an einen magnetischen Bead oder ein magnetisches Partikel gebunden ist, Hydrolysieren der flüssigen Probe zum Herstellen eines Hydrolysats, und
Aufreinigen des Hydrolysats mit magnetbasierter Aufreinigung.
wobei die magnetbasierte Aufreinigung weiter die Schritte umfasst:
Ausfällen von Proteinen mit einem Fällungsreagenz,
Inkubieren der magnetischen Beads oder magnetischen Partikel mit den ausgefällten Proteinen für eine Zeit, die ausreichend ist, um eine Suspension zu erzeugen, und
magnetisches Trennen der magnetischen Beads oder magnetischen Partikel von der Suspension, um einen Überstand zu erzeugen.

2. Verfahren nach Anspruch 1, wobei das Fällungsreagenz ausgewählt ist aus der Gruppe bestehend aus Zinksalzen, Zinksulfat, Glykolen, Alkoholen, Säuren, Sulfaten, Säuren, Acetonitril, und Kombinationen davon.

3. Verfahren nach einem der vorstehenden Ansprüche, wobei die magnetbasierte Aufreinigung auf Immunaufreinigung, Affinitätsaufreinigung oder Ionenaustausch basiert.

4. Verfahren nach Anspruch 3, wobei die magnetbasierte Aufreinigung auf Immunaufreinigung oder Affinitätsaufreinigung basiert und der magnetische Bead oder das magnetische Partikel einen monoklonalen Antikörper, einen polyklonalen Antikörper, ein synthetisches Antikörpermimetikum, ein Aptamer, ein Affimer, DARPins, oder Oligonukleotide oder Peptide umfasst, die mit hoher Affinität an spezifische Targets binden; oder wobei die magnetbasierte Aufreinigung auf Ionenaustausch basiert und die magnetischen Beads oder magnetischen Partikel eine Oberflächenbeschichtung aufweisen, ausgewählt aus der Gruppe bestehend aus Dextran, Natriumcarboxylat, Phosphat, Diphosphat, Polyacrylsäure, Ölsäure, Silica, 2-Hydroxypropyltrimethylammoniumchlorid, Polyethylenimin, Diethylaminoethylcellulose, Poly(4-vinylpyridin), und Natriumsulfonat.

5. Verfahren nach einem der vorstehenden Ansprüche, wobei das Hydrolyseenzym β-Glucuronidase ist.

6. Verfahren nach einem der vorstehenden Ansprüche, wobei das Hydrolyseenzym Codein-6-glucuronid- und Morphin-6-glucuronid-Bindungen hydrolysieren kann.

7. Verfahren nach einem der Ansprüche 2-6, weiter umfassend Aliquotieren des Überstands, wobei (a) der aliquotierte Überstand unter Verwendung von einem Chromatographieinstrument, Microflow, Festphasenextraktion oder einem Trap-and-Elute-Workflow getrennt und/oder angereichert wird; und/oder (b) der aliquotierte Überstand akustisch in ein Open Port Interface injiziert und zu einer Ionenquelle übertragen oder direkt in eine Ionenquelle injiziert wird und der ionisierte Überstand wahlweise mit einem Massenspektrometer analysiert wird.

8. Verfahren nach Anspruch 7, wobei der aliquotierte Überstand akustisch in ein Open Port Interface injiziert und zu einer Ionenquelle übertragen oder direkt in eine Ionenquelle injiziert wird und Ionen von Interesse vor dem Analysieren des ionisierten Überstands mit dem Massenspektrometer unter Verwendung von differentieller Mobilitätsspektrometrie aus dem ionisierten Überstand ausgewählt werden.

9. Verfahren nach einem der vorstehenden Ansprüche, wobei das Verfahren verwendet wird, um eine flüssige Probe für eine klinische Analyse herzustellen, und wobei die klinische Analyse wahlweise verwendet wird, um auf Missbrauchsdrogen zu screenen.

10. Verfahren nach einem der vorstehenden Ansprüche, wobei die magnetischen Beads oder magnetischen Partikel einen monoklonalen Antikörper, einen polyklonalen Antikörper, ein synthetisches Antikörpermimetikum, ein Aptamer, ein Affimer, DARPins oder Oligonukleotide oder Peptide umfassen, die mit hoher Affinität an spezifische Targets binden, oder der magnetische Bead oder das magnetische Partikel Streptavidin umfasst und das Hydrolyseenzym Biotin umfasst.

## Revendications

1. Procédé de préparation d'un échantillon liquide, comprenant :
un traitement de l'échantillon liquide avec une enzyme d'hydrolyse, qui est capable d'hydrolyser des liaisons glycosidiques et est liée à une bille magnétique ou à une particule magnétique, une hydrolyse de l'échantillon liquide pour préparer un hydrolysat, et
une purification de l'hydrolysat avec une purification à base magnétique.
dans lequel la purification à base magnétique comprend en outre les étapes de :
précipitation de protéines avec un réactif précipitant,
incubation des billes magnétiques ou des particules magnétiques avec les protéines précipitées pendant un temps suffisant pour produire une suspension, et
séparation magnétique des billes magnétiques ou des particules magnétiques de la suspension pour produire un surnageant.

2. Procédé selon la revendication 1, dans lequel le réactif précipitant est sélectionné parmi le groupe constitué de sels de zinc, sulfate de zinc, glycols, alcools, acides, sulfates, acides, acétonitrile, et combinaisons de ceux-ci.

3. Procédé selon l'une quelconque des revendications précédentes, dans lequel la purification à base magnétique est basée sur une immunopurification, une purification d'affinité ou un échange d'ions.

4. Procédé selon la revendication 3, dans lequel la purification à base magnétique est basée sur une immunopurification ou une purification d'affinité et la bille magnétique ou la particule magnétique comprend un anticorps monoclonal, un anticorps polyclonal, un mimétique d'anticorps synthétique, un aptamère, un Affimer, des DARPins, ou des oligonucléotides ou peptides qui se lient à des cibles spécifiques avec une forte affinité ; ou dans lequel la purification à base magnétique est basée sur un échange d'ions et les billes magnétiques ou les particules magnétiques présentent un revêtement de surface sélectionné parmi le groupe constitué de dextrane, carboxylate de sodium, phosphate, diphosphate, acide polyacrylique, acide oléique, silice, chlorure de 2-hydroxypropyl triméthylammonium, polyéthylénimine, cellulose diéthylaminoéthyle, poly(4-vinylpyridine), et sulfonate de sodium.

5. Procédé selon l'une quelconque des revendications précédentes, dans lequel l'enzyme d'hydrolyse est la β-glucuronidase.

6. Procédé selon l'une quelconque des revendications précédentes, dans lequel l'enzyme d'hydrolyse est capable d'hydrolyser des liaisons codéine-6-glucuronide et morphine-6-glucuronide.

7. Procédé selon l'une quelconque des revendications 2 à 6, comprenant en outre une mise en aliquotes du surnageant, dans lequel (a) le surnageant mis en aliquotes est séparé et/ou enrichi au moyen d'un instrument de chromatographie, à microdébit, extraction en phase solide, ou d'un protocole de piégeage et d'élution ; et/ou (b) le surnageant mis en aliquotes est injecté acoustiquement dans une interface à port ouvert et transféré vers une source d'ionisation ou directement injecté dans une source d'ionisation, et le surnageant ionisé est facultativement analysé avec un spectromètre de masse.

8. Procédé selon la revendication 7, dans lequel le surnageant mis en aliquotes est injecté acoustiquement dans une interface à port ouvert et transféré vers une source d'ionisation ou directement injecté dans une source d'ionisation, et des ions d'intérêt sont sélectionnés à partir du surnageant ionisé en utilisant une spectrométrie de mobilité différentielle avant d'analyser le surnageant ionisé avec le spectromètre de masse.

9. Procédé selon l'une quelconque des revendications précédentes, dans lequel le procédé est utilisé pour préparer un échantillon liquide pour une analyse clinique, et facultativement dans lequel l'analyse clinique est utilisée pour dépister des stupéfiants.

10. Procédé selon l'une quelconque des revendications précédentes, dans lequel les billes magnétiques ou les particules magnétiques comprennent un anticorps monoclonal, un anticorps polyclonal, un mimétique d'anticorps synthétique, un aptamère, un Affimer, des DARPins, ou des oligonucléotides ou peptides qui se lient à des cibles spécifiques avec une forte affinité ou la bille magnétique ou la particule magnétique comprend de la streptavidine et l'enzyme d'hydrolyse comprend de la biotine.
